# EUROPEAN PATENT APPLICATION

(11) **EP 3 150 234 A1**
(43) Date of publication of application: **05.04.2017**
(21) Application number: 16196419.2
(22) Date of filing: 05.10.2010
(51) Int. Cl.: A61L 15/44, A61L 15/60, A61L 15/46

(54) **WOUND COVERING COMPRISING OCTENIDINE DIHYDROCHLORIDE FOR USE IN THE ANTISEPSIS OF CATHETER INSERTION POINTS**

(30) Priority: 15.10.2009 DE 102009049506
(62) Divisional of application: 10186489.0
(71) Applicant: L'AIR LIQUIDE, SOCIETE ANONYME POUR L'ETUDE ET L'EXPLOITATION DES PROCEDES GEORGES CLAUDE, 75007 Paris (FR); SCHÜLKE & MAYR GmbH, 22851 Norderstedt (DE)
(72) Inventor: BEHRENDS, Sabine, 25482 APPEN (DE); BAUR, Boris, 22307 HAMBURG (DE); DETTMANN, Andreas, 22175 HAMBURG (DE)
(74) Representative: Conan, Philippe Claude

(57) **Abstract**

A wound covering is described which comprises a) a transparent film and b) applied to the film, a transparent hydrogel which comprises octenidine dihydrochloride a thickener and polyol. The wound covering is suitable in particular for use in the antisepsis of catheter insertion points. The active ingredient octenidine dihydrochloride is released from the hydrogel quickly, but in a long-lasting manner.

## Description

The invention relates to a wound covering for use in the antisepsis of catheter insertion points. The wound covering comprises a transparent film and, applied to the film, a transparent hydrogel which comprises octenidine dihydrochloride. It also relates to a wound covering set and a hydrogel comprising octenidine dihydrochloride for use for the antisepsis of catheter insertion points.

To prevent catheter-associated infections, it is usually customary to disinfect and to thereby care for the skin prior to positioning the catheter, to position the catheter and then to cover the catheter insertion point with a customary wound dressing. Alternatively, the insertion point can be covered with a wound dressing which is antimicrobially finished. One example of an antimicrobially finished wound covering is the commercial product biopatch^{®}, which is supplied by Johnson & Johnson and comprises chlorhexidine gluconate as antimicrobial active ingredient. Another known wound covering is the product SilvaSorb Site^{®} from Medline which is antimicrobially finished with silver.

Tietz et al. (Infect Control Hosp Epidemiol. 2005, August, 26 (8): 703-7) describe the use of octenidine dihydrochloride for the care of venous catheter insertion points in patients with a severely weakened immune system.

However, it has been found that when using a wound dressing and also when using a wound covering, on account of the lack of transparency of the carrier material, catheter insertion points are difficult to assess visually and there is therefore the risk that inflammation arises that has become protracted due to it being unnoticed. Added to this is the high rate of incompatibility reactions when using chlorhexidine gluconate (especially in sensitive patients, such as e.g. patients with weakened immune system, oncologic patients) or silver as active ingredient. Furthermore, the specified wound coverings have to be covered with an additional (adhesive) dressing or a film in order to ensure the greatest possible freedom from germs of the catheter insertion point. Added to this, for both of the described methods in the prior art, is an inadequate antimicrobial effect over a prolonged period, which makes it necessary, but undesirable, to frequently change the wound covering/the wound dressing in order to be able to reliably exclude an infection. Such a change is always associated with the risk of a new infection of the catheter insertion point.

On account of these disadvantages, a tending of catheter insertion points that avoids infections has hitherto not been satisfactorily possible.

The object of the present invention was therefore to provide means for preventing catheter-associated infections, with the help of which the described problems of the prior art can be avoided.

Surprisingly, it has now been found that this object is achieved by a wound covering which comprises:
a) a transparent film and
b) applied to the film, a transparent hydrogel which comprises octenidine dihydrochloride.

The invention is based inter alia on the fact that it has been found that hydrogels release the antimicrobial active ingredient octenidine dihydrochloride not only quickly, but surprisingly also in a long-lasting manner. This avoids the incompatibility reactions which are observed with chlorhexidine gluconate or silver as active ingredient. In addition, infections are reliably and long-lastingly prevented. Consequently, in the field of skin antisepsis at an insertion point, it is possible e.g. to bring about increased performance and also an increase in safety compared with customary skin antisepsis. Moreover, the transparent film and the transparent hydrogel permit good monitoring of the catheter insertion point, as a result of which it is possible to avoid an inflammation thereof that has become protracted due to it being unnoticed without a frequent change of the wound covering being necessary for checking.

### I. Wound covering

### a) Film

The wound covering according to the invention has a transparent film. The film does not have to be completely transparent. For example, it suffices if the part of the film covering the catheter insertion point upon subsequent use is transparent. However, the wound covering is preferably transparent not only in the area which has the hydrogel, but also in the adhesive area and especially over the entire area.

The transparency or permeability to light is measured in accordance with ASTM-D 1003-77. According to this, the film can be an opaque to translucent film, but preferably a transparent film. Within the context of the present invention, "transparent film" means a film, the permeability to light of which in accordance with ASTM-D 1003-77 is at least 90%, preferably at least 95%.

The film used according to the invention can be formed as one or more layers, but is preferably formed as one layer. Here, in all embodiments of the invention, preference is given to rectangular films. Typical dimensions of the film are about 3 x about 3 cm. Alternatively, it is possible to design the films according to the invention round in shape; the diameter is then preferably about 3 cm.

A typical thickness of the film used according to the invention is 5-500 µm, preferably 50-200 µm.

Suitable film materials are polymer films, preferably with breathable properties, e.g. polyester polyurethanes or polyether polyurethanes (such as e.g. Estane^{®}, B.F. Goodridge), polyether polyamides (such as e.g. PEBAX^{®}, Arkema), but also hydrophilic and hydrophobic polyurethanes or those with elastomeric properties (such as e.g. HYTREL^{®}, DuPont).

The film preferably has a particularly good water vapour permeability of > 500 g/m²/24 h, but especially of > 2500 g/m²/24 h. The water vapour permeability is determined in accordance with DIN EN 13726-2: 2002.

In this connection, one embodiment is possible in which the film of the wound covering is coated in one area with an adhesive, for example based on poly(meth)acrylate, and is provided in another area with the hydrogel. The area finished with the hydrogel constitutes preferably 5 to 95%, preferably 50 to 80%, such as about 70%, of the total area of the wound covering.

The wound covering according to the invention has a peelable film particularly preferably on the side of the film which has the hydrogel. Peelable films of this type are known from the prior art. For example, peelable films made of polyethylene terephthalalate, polyethylene, polypropylene or polyvinyl chloride are used. This thus gives rise, in the areas of the wound covering which have the hydrogel, to a layer sequence film-hydrogel-peelable film, and in the areas which do not have hydrogel, to a layer sequence film-peelable film.

### b) Hydrogel

The hydrogel used according to the invention is transparent and skin-friendly.

The transparency or permeability to light is measured in accordance with ASTM-D 1003-77. Accordingly, the hydrogel may be an opaque to translucent gel, but is preferably a transparent gel. Within the context of the present invention, "transparent gel" means a gel, the permeability to light of which in accordance with ASTM-D 1003-77 is at least 90%, preferably at least 95%.

Preferably, the hydrogel is sterile. In one preferred embodiment, it comprises, besides
b1) octenidine dihydrochloride, also
b2) thickener,
b3) polyol and
b4) water,
and preferably consists of the components b1) to b4).

Typically, the hydrogel is applied to the film in a thickness of 0.1-5 mm.

### b1) Octenidine dihydrochloride

Octenidine dihydrochloride corresponds to the following formula:

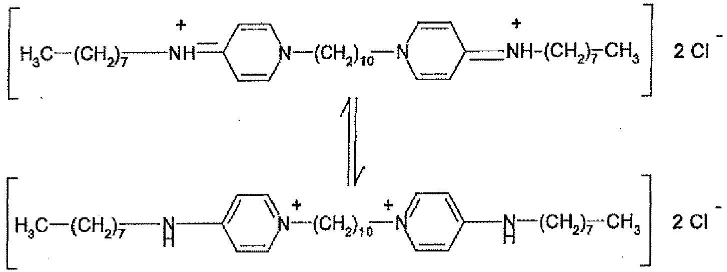

The active ingredient is used successfully by Schülke & Mayr GmbH, Norderstedt, Germany inter alia in the commercial products Octenisept^{®} (wound and mucosa antisepsis), Octeniderm^{®} (preoperative skin antiseptic), Octenidol^{®} (antimicrobial mouth rinse solution) and Octenisan^{®} (antimicrobial washing lotion). Octenidine dihydrochloride has a wide antimicrobial spectrum of activity. According to the invention, it has been found that the active ingredient octenidine dihydrochloride is particularly advantageously released from a hydrogel, as demonstrated by the examples.

Preferably, the amount of octenidine dihydrochloride in the hydrogel is 0.001 to 1% by weight, more preferably 0.005 to 0.5% by weight, such as 0.01 to 0.2% by weight and in particular 0.05 to 0.1 % by weight.

### b2) Thickener

Thickener is present in the hydrogel preferably in an amount of from 0.5 to 10% by weight, preferably 1 to 5% by weight, more preferably 1.5 to 4% by weight and in particular 2 to 3% by weight.

Preferred thickeners are xanthan gums, thickening silicas, polyvinyl alcohols, polyacrylates, gelatin, pectin, casein, agar agar, starch, tragacanth, polyvinylpyrrolidone, cellulose and cellulose derivatives such as hydroxyethylcellulose, and mixtures thereof.

### b3) Polyol

A preferred amount of polyol in the hydrogel is 0.1 to 20% by weight.

Exemplary polyols are selected from di-, tri- and tetraols, preferably polyethylene glycol and/or glycerol.

Particularly preferred hydrogels according to the invention and their preparation are given below:

### Hydrogel I (data in percent by weight)

0.05% octenidine dihydrochloride
9.90% propylene glycol
2.50% hydroxyethylcellulose 4000
87.55% demineralized (dem.) water

This exemplary hydrogel is prepared as follows: water is introduced as initial charge and propylene glycol and octenidine dihydrochloride are dissolved therein with heating to 60°C to give a clear solution. The hydroxyethylcellulose is then added and stirring is carried out until the distribution is homogeneous. The product is then left to swell overnight.

### Hydrogel II (data in percent by weight)

0.10% octenidine
2.85% glycerol 85%
94.55% dem. water
2.50% hydroxyethylcellulose 4000

### Hydrogel III (data in percent by weight)

0.10% octenidine dihydrochloride
2.00% phenoxyethanol
0.50% glycerol 85%
0.40% sodium gluconate
1.00% cocamidopropylbetaine 30%
2.75% hydroxyethylcellulose 4000
93.25% dem. water

This exemplary hydrogel is prepared as follows: water is introduced as initial charge, then the constituents octenidine dihydrochloride, phenoxyethanol, glycerol, sodium gluconate and cocamidopropylbetaine are added. The mixture is then stirred until a clear solution is formed. The cellulose is then stirred in homogeneously. Finally, the mixture is left to swell overnight.

The hydrogel can also comprise one or more antiseptic active ingredients and/or functional additives.

Examples of further antiseptic active ingredients are chlorhexidine salts, such as chlorhexidine digluconate, polyhexanide salts, silver or silver salts, lower alcohols such as ethanol, isopropanol, n-propanol or mixtures thereof.

Examples of functional additives are humectants (such as urea, glyceryl ethers such as 1-(2-ethylhexyl)glycerol ether (Sensiva^{®} SC 50), sorbitol, collagens, plant extracts, e.g. aloe vera, hyaluronic acid, glycerol), osmolytes (such as sodium chloride, Ringer's solution, carbohydrates, proteins), buffers or pH regulators (e.g. acids, alkalizing agents, citrates, phosphates), complexing agents such as EDTA, phosphates, polyphosphates, 8-hydroxyquinoline, antiinflammatory additives such as allantoin, plant extracts such as camomile or aloe vera, bisabolol, dexpanthenol, medicament active ingredients, hemistatic additives, antioxidants (such as vitamin E or derivatives, BHA, BHT, vitamin C, gallates, acetylcystein, 3-tert-butyl-4-hydroxyanisole, 2,6-di-tert-butyl-p-cresol, tert-butylhydroquinone, caffeic acid, chlorogenic acid, cysteine, cysteine hydrochloride, decyl mercaptomethylimidazole, diamylhydroquinone, di-tert-butylhydroquinone, dicetyl thiodipropionate, digalloyl trioleate, dilauryl thiodipropionate, dimyristyl thiodipropionate, dioleyl tocopherol methylsilanol, disodium rutinyl disulphate, distearyl thiodipropionate, ditridecyl thiodipropionate, dodecyl gallate, erythorbic acid, ethyl ferulate, ferulic acid, hydroquinone, p-hydroxyanisole, hydroxylamine hydrochloride, hydroxylamine sulphate, isooctyl thioglycolate, cojic acid, madecassicoside, methoxy-PEG-7-rutinyl succinate, nordihydroguaiaretic acid, octyl gallate, phenylthioglycolic acid, phloroglucinol, propyl gallate, rosmarinic acid, rutin, sodium erythorbate, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocophersolan, tocopherol (e.g. vitamin E) and its derivatives (e.g. vitamin E derivatives such as vitamin E acetate, vitamin E linoleate, vitamin E nicotinate and vitamin E succinate), o-tolylbiguanide, tris(nonylphenyl) phosphite, dexpanthenol, alpha-hydroxycarboxylic acids (e.g. glycolic acid, lactic acid, mandelic acid) and salts thereof, p-hydroxybenzoic acid esters (e.g. methyl, ethyl, propyl or butyl esters thereof), dimethyloldimethylhydantoin, n-acylamino acids and salts thereof (e.g. N-octanoylglycine, Lipacid C8G), ascorbic acid and hinokitiol), preservatives such as phenoxyethanol, propylene glycol, parabens, ascorbic acid, benzoates, thiazolinones, ethylhexylglycerol, sorbic acid and salts thereof, solvents such as lower alcohols, aromatic alcohols such as benzyl alcohol, phenoxyethanol, anaesthetics such as benzyl alcohol, electrolytes such as proteins, irons (sodium, chloride, magnesium, calcium), viscosity modifiers, active ingredient boosters such as glycerol ethers (e.g. Sensiva SC 50), skincare additives such as allantoin, Sensiva SC 50, dexpanthenol, stabilizers such as vitamin E, BHA, BHT, wetting agents such as Sensiva SC 50, macrogol lauryl ether (e.g. Brij 35), macrogol glycerol hydroxystearate (Cremophor RH 40), betaines, fatty alcohol polyalkoxylates, sorbitan fatty acid esters (Tween) and polyoxyethylene sorbitan fatty acid esters, alkyl glycosides, EO/PO block copolymers, skin-cooling additives such as menthyl glycerol ether, dyes, marking agents, adhesion improvers, aroma substances and fragrances.

The viscosity of the hydrogels used according to the invention is typically at least 20 000-500 mPa*s, preferably 15 000-1000 mPa*s, particularly preferably 10 000-3000 mPa*s, measured by measurement, corresponding to DIN 53019, of viscosities and flow curves using rotary viscometers.

The pH of the hydrogels used according to the invention is generally in the pH range from 2 to 10, preferably in the pH range from 3 to 8, particularly preferably in the pH range from 4 to 7.

### II. Hydrogel for the antisepsis of catheter insertion points

The invention also relates to a hydrogel which comprises octenidine dihydrochloride for use for the antisepsis of catheter insertion points.

### III. Wound covering set

The invention also relates to a wound covering set which comprises
a) a transparent film and
b) a transparent hydrogel which comprises octenidine dihydrochloride.

In this embodiment, the transparent film and the transparent gel are present separately, for example in a kit. It is thus possible to firstly place the hydrogel on the catheter insertion point and then to cover the catheter insertion point with the transparent film. The application of the hydrogel can take place as a result of application by means of swabs, patches or spraying methods. Alternatively, it is possible to place the hydrogel on the film prior to application and then to cover the catheter insertion point with the film finished with the hydrogel.

The wound covering according to the invention can be used, for example, for covering central venous catheters (CVC) and for covering other peripheral accesses.

Advantages of the use according to the invention of a hydrogel comprising octenidine dihydrochloride are:
- broad and rapid-onset microbicidal effectiveness
- long-lasting microbicidal effectiveness
- very good to adequately good release of the active ingredient from the hydrogel
- very good stability of the active ingredient and of the hydrogel
- very good thermal stability of the active ingredient and of the hydrogel
- heat-sterilizable
- clear and/or translucent and/or transparent, even after prolonged storage under unfavourable storage conditions
- good adhesion to skin, mucosa and medical articles such as catheters
- good spreadability on surfaces
- very good and rapid wetting of surfaces
- very good skin compatibility, very good compatibility on injured skin, very slight cytotoxic effect
- pleasant skin feel, possibly skin-cooling effect
- free from undesired by-products or degradation products
- can be washed off easily, can be removed without residues
- material-conserving
- economical
- high acceptance among medical personnel and patients.

The advantages of the invention arise in particular from the following examples.

### Examples

### Method:

### Bioavailability of octenidine dihydrochloride in a hydrogel

The bioavailability of octenidine dihydrochloride from a hydrogel was tested using the Sartorius liberation model (type SM 16755).

The ointment liberation cell consists of three see-through Plexiglas plates held together by bolts and nuts. Chambers are built into two plates. Heating water passes through the heating space in the middle plate. In the same plate, there is, opposite the heating space, the ointment chamber for accommodating the substance under investigation. In the plate, the acceptor medium flows through the ripple plate, where it comes into contact with the membrane of the released active ingredient.

Temperature medium and acceptor medium thus have two independent cycles. The sample for determining the content is drawn from the acceptor medium chamber.

### Test product: Hydrogel I

Photometer method: Photometer Cadas 100, Dr. Lange (280 nm)

A calibration for the active ingredient octenidine dihydrochloride is firstly carried out.

The test product is weighed into the ointment chamber. Samples are taken at the specified times. The removed sample volume is replaced completely by new acceptor medium. At the end of the total runtime, the samples are measured at 280 nm in 1 cm quartz cuvettes.

The results are shown in the table below:

| Time (h) | Released amounts of octenidine dihydrochloride (in % of the total amount) |
|---|---|
| 0.5 | 10.83 |
| 1 | 16.73 |
| 2 | 27.54 |
| 3 | 36.22 |
| 4 | 43.15 |
| 5 | 50.27 |
| 6 | 56.06 |
| 8 | 65.31 |
| 24 | 90.04 |

The results show that a good and continuous release of the active ingredient octenidine dihydrochloride from the hydrogel takes place. After 24 hours, 90% of the contained active ingredient have been released from the gel.

## Claims

1. Wound covering comprising
a) a transparent film having a thickness of 5-500µm and a vapor permeability greater than 500g/m²/24h,
b) applied to the film in a thickness of 0.1-5mm, a transparent hydrogel, the permability to light of which being at least 90%, which comprises
b1) 0.001 to 1% by weight octenidine dihydrochloride,
b2) 0.5 to 10% of a thickner selected from xanthan gums, thickening silicas, polyvinyl alcohols, polyacrylates, gelatins, pectin, casein, agar agar, starch, tragacanth, polyvinylpyrrolidone, cellulose and cellulose derivatives,
b3) 0.1 to 20% by weight of polyol selected from selected from di-, tri- and tetraols, and mixtures thereof,
b4) water.

2. Wound covering according to claim 1, **characterized in that** the hydrogel comprises 0.005 to 0.5% by weight, of octenidine dihydrochloride.

3. Wound covering according to claim 2, **characterized in that** the hydrogen comprises 0.01 to 0.2% by weight of octenidine dihydrochloride.

4. Wound covering according to claim 3, **characterized in that** the hydrogen comprises 0.05 to 0.1 % by weight, of octenidine dihydrochloride.

5. Wound covering according to claim 1 or claim 2, **characterized in that** the hydrogel comprises 1 to 5% by weight, of thickener.

6. Wound covering according to claim 5, **characterized in that** the hydrogel comprises 1.5 to 4% by weight of thickener.

7. Wound covering according to claim 6, **characterized in that** the hydrogel comprises 2 to 3% by weight, of thickener.

8. Wound covering according to one of Claims 1 to 7, **characterized in that** the thickener is hydroxyethylcellulose.

9. Wound covering according to one of Claims 1 to 8, **characterized in that** the olyol is propylene glycol and/or glycerol.

10. Wound covering according to one of Claims 1 to 9, **characterized in that** the hydrogel also comprises one or more antiseptic active ingredients and/or functional additives.

11. Wound covering according to Claim 10 **characterized in that** the hydrogel consists in
0.10% octenidine dihydrochloride
2.00% phenoxyethanol
0.50% glycerol 85%
0.40% sodium gluconate
1.00% cocamidopropylbetaine 30%
2.75% hydroxyethylcellulose 4000
93.25% demineralized water.

12. Wound covering according to one of Claims 1 to 9, **characterized in that** the hydrogel consists of components b1) to b4).

13. Wound covering according to Claim 12 **characterized in that** the hydrogel consists in
0.05% octenidine dihydrochloride
9.90% propylene glycol
2.50% hydroxyethylcellulose 4000
87.55% demineralized water.

14. Wound covering according to Claim 12 **characterized in that** the hydrogel consists in
0.10% octenidine
2.85% glycerol 85%
94.55% demineralized. water
2.50% hydroxyethylcellulose 4000.
